(19) Europäisches Patentamt
European Patent Office
Office européen des brevets

(11) Veröffentlichungsnummer: **0 134 552 B1**

# (12) EUROPÄISCHE PATENTSCHRIFT

(45) Veröffentlichungstag der Patentschrift: **28.10.92**

(21) Anmeldenummer: **84109600.1**

(22) Anmeldetag: **13.08.84**

(51) Int. Cl.5: **C12N 5/00**, C12P 21/00, C07K 3/02, G01N 33/577

## (54) Monoklonaler Antikörper mit hoher Affinität zum Digoxin.

(30) Priorität: **20.08.83 DE 3330160**

(43) Veröffentlichungstag der Anmeldung:
**20.03.85 Patentblatt 85/12**

(45) Bekanntmachung des Hinweises auf die Patenterteilung:
**28.10.92 Patentblatt 92/44**

(84) Benannte Vertragsstaaten:
**AT BE CH DE FR GB IT LI LU NL SE**

(56) Entgegenhaltungen:
**EP-A- 0 044 441**

**THE JOURNAL OF IMMUNOLOGY, Band 129, Nr. 3, September 1982, Seiten 1165-1172, The American Association of Immunologists, US; MEREDITH MUDGETT HUNTER et al.: "High-affinity monoclonal antibodies to the cardiac glycoside, digoxin"**

(73) Patentinhaber: **BOEHRINGER INGELHEIM KG**
**Postfach 200**
**W-6507 Ingelheim am Rhein(DE)**
(84) Benannte Vertragsstaaten:
**BE CH DE FR IT LI LU NL SE AT**

(73) Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Struck, Carl-Julius, Dr.**
**Menzelstrasse 6**
**W-6200 Wiesbaden(DE)**

EP 0 134 552 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**BIOLOGICAL ABSTRACTS, Band 72, 1981, Seite 4205, Nr. 40323, Philadelphia, PA., US; B.E. BANG et al.: "Studies of monoclonal and polyclonal antidigoxin antibodies for serum digoxin radioimmunoassay", & SCAND. J. CLIN. LAB. INVEST. 41(1): 75-78, 1981**

**BIOCHEMISTRY, Band 22, 1983, Seiten 1153-1158, American Chemical Society, US; J. NOVOTNY et al.: "Amino acid sequence of the light chain variable region from a mouse anti-digoxin hybridoma antibody"**

**FEDERATION PROCEEDINGS, Band 39, Nr. 3, 1980, Seite 928, Nr. 3473; M. MUDGETT-HUNTER et al.: "Hybridoma antibodies to the cardiac glycoside digoxin"**

## Beschreibung

Die Erfindung betrifft einen monoklonalen Antikörper mit hoher Affinität zu dem Digitalisglycosid Digoxin, geringer Sensitivität zu verwandten Glycosiden und Spironolacton, die diesen monoklonalen Antikörper produzierenden Zellinien, Verfahren zu deren Herstellung, die Verwendung dieses Antikörpers sowie ein Testsystem, das diesen monoklonalen Antikörper enthält.

Etwa drei Millionen Coronarpatienten in der Bundesrepublik werden derzeit täglich mit Digitalisglycosiden therapiert. (J.R.OCHS, G.BODEM, Med. Welt 30, 602 (1978)) Damit gehört diese Präparategruppe zu den am häufigsten verschriebenen Arzneimitteln. Innerhalb der gesamten Gruppe spielt Digoxin mit einem Anteil von über 90 % die bedeutendste Rolle.

Die weite Verbreitung und der häufige Gebrauch der Digitalisglycoside darf jedoch nicht darüber hinwegtäuschen, daß es sich hierbei um potentiell gefährliche Wirkstoffe handelt - die therapeutische Breite ist äußerst gering. Im Sinne einer effektiven und sicheren Therapie ist daher die kontinuierliche Überwachung der Digitalis-Spiegel erforderlich. Eine Reihe verschiedener Testverfahren wurde für diesen Zweck entwickelt. Dabei nutzt man die vom Organismus gegen das Glycosid gebildeten Antikörper als Nachweismittel aus. Diese Antikörper gewinnt man aus dem Serum der mit Digitalis immunisierten Wirtszellen. Man erhält auf diese Weise Antiseren polyklonaler Natur; d. h. diese Seren enthalten verschiedene Antikörper.

Ein erhebliches Problem stellt jedoch bei vielen Tests für Digoxin die starke Kreuzreaktion mit dem sich nur durch eine OH-Gruppe in Position 12 von Digoxin unterscheidenden Digitoxin dar:

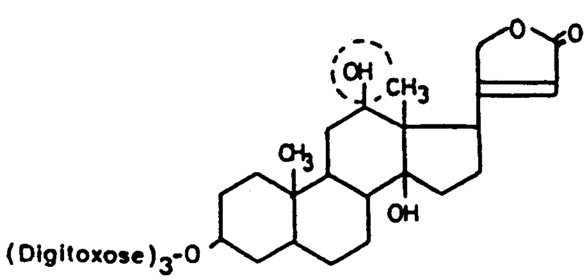

1

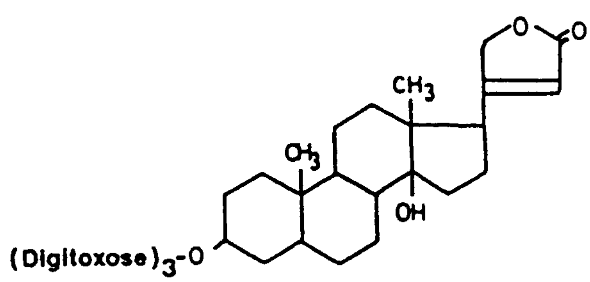

2

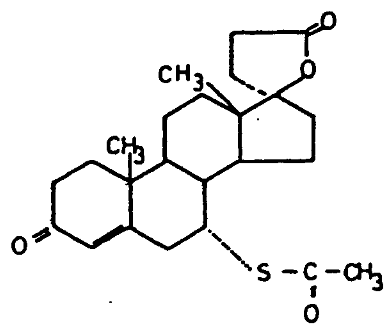

3

Strukturformeln von (1) Digoxin, (2) Digitoxin und (3) Spironolactone

Wesentlich bedeutsamer jedoch als eine Kreuzreaktion durch Digitoxin ist die mögliche, bei vielen - auch kommerziellen - Assays auftretende und störende Interferenz des häufig auch zusammen mit Digoxim verabreichten Aldosteron-Antagonisten Spironolactone (z.B. Aldactone®). Diese Substanz wird in wesentlich höherer Dosierung verabreicht und kann infolgedessen bei ungenügender Diskriminierungsfähigkeit des Tests bedrohlich hohe Digoxinspiegel vortäuschen.

Diese Kreuzaktivitäten der üblicherweise im Kaninchen oder im Schaf erzeugten Antikörper wurden durch aufwendige affinitätschromatographische Reinigungsverfahren zu verbessern versucht. Nachteilig hierbei ist jedoch der Bedarf an großen Antiserummengen (wegen geringer Reinigungsausbeuten) und die Tatsache, daß eine Verbesserung der Kreuzaktivität oft mit einer deutlichen Verminderung der Nachweisempfindlichkeit einhergeht. Dies ist darin begründet, daß gerade die hochaffinen Antikörper, die für eine

hohe Sensitivität verantwortlich sind, nur sehr schwer oder überhaupt nicht von der Affinitätsmatrix zu eluieren sind.

Als Alternative zu herkömmlichen Verfahren der Antikörperproduktion gewinnt seit Ende der siebziger Jahre, ausgehend von den grundlegenden Arbeiten von KÖHLER und MILSTEIN 1975/76 (Nature, 256, 495 (1975))die Produktion von Antikörpern durch Hybridom-Zellinien in der Zellkultur zunehmend an Bedeutung. Diese Zellinien werden nach somatischer Fusion von Milzzellen einer zuvor immunisierten Maus mit Zellen einer Maus-Tumorlinie und wiederholten anschließenden Klonierungen erhalten.

Sie zeichnen sich, da sie jeweils auf eine einzige Elternzelle zurückzuführen sind, dadurch aus, daß sie jeweils ausschließlich nur einen einzigen Antikörpertypus von einheitlicher Spezifität, einen monoklonalen Antikörper (mAK), produzieren. Ihr theoretisch unbegrenztes Wachstum als Tumorzellinie ermöglicht darüberhinaus die Produktion theoretisch unbegrenzter Antikörpermengen.

Für Tests zur ständigen Überwachung Digoxin-behandelter Patienten oder zur Abklärung von Intoxikationen ist gerade wegen der zuletzt genannten typischen Eigenschaften die Verwendung eines mAK im Testsystem sinnvoll und wünschenswert.

HUNTER et al. (J. Immunol., 129, 1165-1172 (1982)) beschreiben monoklonale Antikörper, welche eine höhere Affinität zum Digoxin als zum Digitoxin besitzen (Fig. 2 und Tabelle IV). Jedoch sind die alleinigen Bildner der monoklonalen Antikörper bestimmte unikate Hybridome, welche durch das dort angegebene Herstellungsverfahren nicht identisch wiedergegeben werden können. Ein weiterer Nachteil sind die umfangreichen Behandlungsschritte, die nach der Digoxinselektion durchgeführt werden müssen, bis eine Affinität zum Digitoxin festgestellt werden kann.

Der vorliegenden Erfindung lag daher die Aufgabe zugrunde, eine Mäusehybridzellinie, die einen monoklonalen Antikörper mit höherer Affinität zum Digoxin als zum Digitoxin bildet, zur Verfügung zu stellen, die auf einem neuen und reproduzierbaren Weg hergestellt werden kann.

Antikörper bestehen aus einem konstanten Bereich, der allen Antikörpern gemeinsam ist, und einem variablen Bereich.

Die spezifischen Eigenschaften eines Antikörpers hängen nur von den variablen Bereichen ab. Wird nun ein Organismus von einem Antigen zur Produktion von Antikörpern stimuliert, so entstehen Immunglobuline, die gegen dies Antigen gerichtet sind. Da jedoch ein Antigen üblicherweise mehrere determinante Gruppen, mehrere Haftstellen für Antikörper besitzt, entsteht ein Gemisch von Antikörpern, die zwar alle gegen dasselbe Antigen gerichtet sind, jedoch unterschiedliche Affinitätskonstanten besitzen und unterschiedlich in ihrer Spezifität sind.

Die erfindungsgemäße Aufgabe wurde nun überraschenderweise dadurch gelöst, daß man Mäuse mit Digoxin immunisiert und nach somatischer Zellfusion mit einer Maus-Tumorzellinie aus den dadurch erhaltenen Zellkulturüberständen mit einem Radioimmunoassay (RIA) oder einem ELISA-Assay diejenigen Hybride auswählt, die monoklonale Antikörper mit höherer Affinität zu Digoxin als zu Digitoxin bilden.

Dazu wurden gleiche Proben aus gleichen Zellkulturüberständen auf ihre Aktivität gegenüber Digoxin einerseits und Digitoxin andererseits mittels eines Radioimmunoassays untersucht. Durch direkten Vergleich dieser Aktivitäten untereinander konnten die Zellinien ausgewählt werden, die zwar hoch aktive mAK gegenüber Digoxin, jedoch schwach aktive mAK gegenüber Digitoxin produzierten. Diese hochspezialisierten Hybridzellen wurden isoliert und etabliert.

Die Immunisierung der Mäuse, Isolierung der Milzzellen, Fusionierung der Zellen, Kultivierung und Selektionierung der Hybride, Subklonierung der Hybride, die den gesuchten Antikörper produzieren, die Isolierung der Hybride und der Antikörper wird nach dem Fachmann bekannten Verfahren durchgeführt. Es sei beispielsweise auf die Arbeiten von KÖHLER und MILSTEIN, Nature 256, 495 (1975), HOCHKEPPEL et al. Eur. J. Biochem. 118, 437 (1981) und SECHER et al., Nature 285, 446 (1980) hingewiesen.

Für die Immunisierung stehen dem Fachmann die üblicherweise verwendeten Balb/c-Mäuse zur Verfügung.Zur Stabilisierung und Vermehrung der Hybride kann man in vitro und in vivo kultivieren. Bei der in vivo-Kultur injiziert man die Hybride in die Bauchhöhle von zuvor mit einem Tumorstimulator behandelten Mäusen. Die vermehrten monoklonalen Antikörper der gewünschten Spezifität lassen sich dann aus der Ascites-Flüssigkeit isolieren und gegebenenfalls reinigen.

Es zeigte sich, daß dieser erfindungsgemäße monoklonale Antikörper eine hohe Affinität zu Digoxin aufweist; damit ist eine wesentliche Bedingung für einen Einsatz im Testsystem erfüllt.

Im Gegensatz zu den meisten herkömmlichen anti-Digoxin-Seren polyklonaler Natur und auch zu mehreren anderen in der Literatur beschriebenen monoklonalen anti-Digoxin-Antikörpern (YELTON, D.E., SCHARFF, M.D.; Ann. Rev. Biochem. 50, 657 - 680, (1981); MARGOLIES, N.M., HUNTER, M.M.; SMITH, T.W.; NOVOTNY, J.; HABER E.; in: Monoclonal antibodies and T-cell hybridomas; HÄMMERLING, G.J.; HÄMMERLING, U.; KEARNEY, J.F.; eds.; pp 367 - 374 Elsevier/North Holland (1981); BANG, B.E.; HURME, M.; IUNTUNEN,K.; MÄKELÄ O.; Scand. J. clin. Lab. Invest. 41, 75 - 78 (1981); HUNTER, M.M.; MARGOLIES, M.N. JU, A.; HABER, E.; J. Immunol., 129, 1165 - 1172 (1982)) weist der erfindungsgemäße mAK eine ausgeprägte Spezifität für Digoxin auf. Selbst eine dem Digoxin so ähnliche Substanz wie Digitoxin kann deutlich unterschieden werden (Kreuzreaktion ca. 1,3 %).

Daß für andere ähnlich strukturierte Substanzen, z.B. aus der Gruppe der Steroide, praktisch keine Kreuzreaktion feststellbar ist, konnte bestätigt werden. Einem Einsatz dieses mAK bei der Therapieüberwachung kommt besonders die äußerst geringe Affinität zu dem häufig parallel zu einer Digoxin-Therapie verabreichten Aldosteron-Antagonisten Spironolactone zugute; es war hier nur eine vernachläßigbar geringe Kreuzreaktion von weniger als 0,007 % festzustellen.

Auf der Basis des monoklonalen anti-Digoxin-Antikörpers wurde ein Test-System in Microtiter®-Platten aufgebaut,das es ermöglicht, neben einer deutlichen Einsparung an Arbeitszeit, auch die Menge der benötigten Reagenzien deutlich zu reduzieren. Insbesondere die Ausführung als "micro-RIA"*erlaubt aufgrund seiner Sensitivität (Nachweisgrenze: 0,8 ng/ml) Messungen von unvorbehandelten Plasmaproben nach Gabe therapeutischer Dosen von Digoxin.

Insgesamt übertrifft das auf dem erfindungsgemäßen mAK basierende Testsystem in Bezug auf Sensitivität und Spezifität die derzeit kommerziell erhältlichen Kits.

Das micro-Testsystem macht eine Verwendung als Routineassay möglich und sinnvoll. Ein besonderer Vorteil eines auf einem mAK aufgebauten RIA ist hierbei, daß man über einen theoretisch unbegrenzten Zeitraum hinweg auf eine Antikörperquelle zurückgreifen könnte, die als kontinuierlich wachsende Zellinie monoklonale Antikörper mit gleichbleibenden Eigenschaften in theoretisch unbegrenzter Menge produzieren kann, sei es in der Suspensionszellkultur oder in der Ascitesmaus.

Legende zu den Abbildungen im Anhang 2:

- Fig. 1: Zunahme der Antikörperproduktion für zwei Tiere.
- Fig. 2: mikroskopisches Bild der Zellklone nach 10 Tagen Wachstum.
- Fig. 3: Elutionsprofil von Ascitesflüssigkeit bei affinitätschromatographischer Reinigung (über DEAE Affi Gel Blue®.
- Fig. 4: Empfindlichkeit des Digoxin-RIA bei Verwendung des mAK D 50 ("micro-RIA"-System). Mittelwerte (± Standardabweichung) aus einer Vierfachbestimmung (mAK-Verd. 1 : 40.000 T = 6.000 cpm, bo = 40 %, NSB = 1,7 %).
- Fig.5: Interferenz strukturähnlicher Substanzen im Digoxin-micro-RIA.
- Fig. 6: Ablaufschema des "micro-RIA". Die einzelnen Arbeitsschritte sind in Kap. 1.9.2 (Methodik) ausführlich beschrieben. Zusammenfassung: 100 µl Probe bzw. Digoxin-Standard werden ebenso wie 100 µl $^{125}$J-Digoxin und 100 µl anti-Digoxin mAK in die Vertiefungen einer Microtiter®-Platte pipettiert. Nach Inkubation bei Raumtemperatur erfolgt die Trennung der gebundenen und freien Radioaktivität durch Dextran-Aktivkohle. Die gebundene Radioaktivität wird anhand von Aliquots der einzelnen Ansätze bestimmt.

Im folgenden wird die Erfindung näher erläutert:

## 1. Methodik

### 1.1 Herstellung des Immunogens

Die Herstellung des Immunogen-Konjugates, bestehend aus Digoxin als Hapten und Rinderserumalbumin (bovine serum albumin, BSA, MILES) als Trägermolekül wird entsprechend der bei BUTLER & CHEN, Proc. Nat. Acad. Sci. (USA) 57, 71-78 (1967) beschriebenen Methode durchgeführt.

### 1.2 Immunisierung der Spendermäuse

Zur Immunisierung werden ausschließlich Mäuse des Stammes Balb/c verwendet, es werden jeweils Gruppen a 20 Tiere immunisiert. Jede Maus erhält intraperitoneal 200 µl einer Emulsion aus Digoxin-BSA und Complete Freund's Adjuvans (CFA, DIFCO Laboratories) im Verhältnis 1:3 (20 - 50 µg Immunogen/Tier).

Nach vier Wochen erfolgt eine Auffrischung (Booster-Injektion) mit 20 - 50 µg Digoxin-BSA in 0.9 % NaCl. Gegebenenfalls wird diese Booster-Injektion in 4-wöchigem Abstand mehrfach wiederholt.

Die Immunreaktion der Mäuse wird anhand regelmäßiger Blutentnahmen (Pl. retroorbitalis) überwacht. Zur Bestimmung des Antikörpertiters im Vollblut werden Reagenzien aus einem kommerziellen RIA-Kit (DIAGNOSTIC PRODUCTS Corp.) verwendet.

* RIA = Radio-Immuno-Assay

1.3 Kultur der Maus-Tumorzellinie

Als Fusionspartner wird die Zellinie X63.AG8-653 *verwendet. Die Zellen werden in Einfriermedium (siehe Anhang 1) bei -196°C in Flüssigstickstoff aufbewahrt. Eine Woche vor Fusion wird ein Aliquot aufgetaut und in Plastic-Petrischalen (10 cm ⌀, GREINER) in RPMI 1640 Zellkulturmedium (s. Anhang 1) ausgesetzt. Zur Fusion werden die Zellen in der logarithmischen Wachstumsphase verwendet.

1.4 Somatische Zellfusion

Die Milz einer immunisierten Maus wird 3 - 4 Tage nach der letzten Booster-Injektion unter sterilen Bedingungen entnommen. Durch vorsichtiges Zerreiben auf einem Edelstahlsieb (Porenweite 100 µm) werden die Milzzellen aus dem Gewebeverband isoliert und in PBS (Phosphat-gepufferte Salzlösung, siehe Anhang 1) aufgenommen. Die Zellen werden zweimal in DPBS**gewaschen (Zentrifugation bei 1000 rpm, 5 min) und anschließend in DPBS aufgenommen. In diesem Ansatz werden Milzzellen und Ag8-Zellen im Verhältnis 2 : 1 gemischt. Durch Zugabe einer Polyäthylenglycol-Lösung (PEG 4000 71 %, DMSO 6 % in Dulbecco's PBS; Lieferanten: ROTH/SIGMA/, SEROMED) wird die Fusion der Zellpopulationen gestartet. Nach 1 min wird das PEG durch Zugabe von Dulbecco's PBS ( DPBS, s. Anhang 1) ausverdünnt. Die Zellsuspension wird bis zur restlosen Entfernung des PEG gewaschen und anschließend mit einer Zelldichte von ca. $10^6$-Zellen/ml in Hypoxanthin/Aminopterin/Thymidin-Selektionsmedium (Littlefield's HAT-Medium, s. Anhang 1) aufgenommen. In diesem Selektionsmedium überleben ausschließlich fusionierte Zellen, nicht jedoch die ebenfalls noch in der Suspension befindlichen unfusionierten Milz- bzw. Ag8-Zellen.

1.5 Kultur von Hybridomzellen

Die Zellsuspension nach Fusion (in HAT-Medium) wird in 200 µl Aliquots in die 96 Kavitäten von Microtiter$^R$-Platten (COSTAR Typ 3596) pipettiert. Bei 37° C und 95 % relativer Luftfeuchtigkeit werden die Kulturen in einer Atmosphäre von 93 % Luft und 7 % $CO_2$ ohne Mediumwechsel 7 - 10 Tage im Begasungsbrutschrank inkubiert. Das Zellwachstum wird in dieser Zeit regelmäßig überwacht.

Nach zwei bis drei Wochen werden mittels eines speziell für diesen Zweck entwickelten Screening-Tests (s.Kap. 1.9) positive, d.h. Immunoglobuline der gewünschten Spezifität produzierende Kulturen identifiziert. Zum gleichen Zeitpunkt beginnt auch die schrittweise Umstellung der Kulturen von HAT-Medium über HT-Medium (s. Anhang 1) auf RPMI 1640-Medium.

1.6 Klonieren von Hybridomzellkulturen

Positive proliferierende Kulturen werden zunächst in größeren Kulturgefäßen expandiert (COSTAR Zellkulturplatten Typ 3524 bzw. Typ 3506). Die anschließende Klonierung erfolgt nach der Methode des "limiting dilution cloning". Hierzu wird die Suspension einer positiven Kultur so weit verdünnt, daß statistisch pro neu angelegter Kultur 10 bzw. 1 Zelle inoculiert wurde. Nach 8 - 12 Tagen werden bereits makroskopisch Kolonien sichtbar, die sich von Einzelzellen herleiten. Um sicherzustellen, daß monoklonale Kulturen wachsen, Kulturen, die sich jeweils von einer einzigen gemeinsamen Elternzelle ableiten, wird der Klonierungsschritt mindestens zweimal durchgeführt.

1.7 Expansion von Hybridom-Zellkulturen

1.7.1 Expansion in der Zellkultur (in vitro)

Etwa $10^3$-Zellen werden in 10 ml RPMI 1640 in Petrischalen (10 cm ⌀, GREINER) ausgesät. Alternativ kommen auch Zellkulturflaschen zur Anwendung. Der zellfreie Überstand enthält nach einigen Tagen die von den Zellen sekretierte mAK. Er kann direkt im RIA eingesetzt werden.

1.7.2 Expansion in der Ascitesmaus (in vivo)

*Kearney et al, J. Immunol. 123, 1548 (1979)

**(Dulbecco's PBS, s.Anhang 1)

Balb/c-Mäuse erhalten zur Konditionierung des Peritoneums 0,5 ml des Mineralöls Pristan® (ROTH) i.p.. Innerhalb eines Zeitraumes von 7 - 60 Tagen wird den so vorbehandelten Tieren eine Suspension von $10^6$ -$10^7$ Hybridomzellen/Tier in PBS i.P. appliziert. Nach 8 - 10 Tagen wird mit einer Kanüle das Peritoneum angestochen und die zellhaltige Ascitesflüssigkeit gesammelt.

Von der entnommenen Ascitesflüssigkeit werden die zellulären Bestandteile durch Zentrifugation abgetrennt (1000 rpm, 10 min). Der Überstand, der auch die monoklonalen Antikörper enthält, wird anschließend in Aliquots (ggf. nach Verdünnung) bei -70° C eingefroren oder der affinitätschromatographischen Reinigung zugeführt.

1.8 Reinigung der Ascitesflüssigkeit

Die Reinigung erfolgt in Anlehnung an die Methode von BRUCK et al. J. Immunol. Meth. 53, 313- 319, (1982)

1.8.1 Vorbehandlung der Ascites-Flüssigkeit

Ascites-Flüssigkeit wird 5 Min. bei 1000 x g zentrifugiert um selluläre Bestandteile zu sedimentieren. Anschließend werden Zellbruchstücke und Fibrin-Aggregate durch Ultrazentrifugation bei 100 000 x g/30 min. abgetrennt. Der Überstand wird anschließend über Nacht gegen das 100-fache Volumen Tris-HCl-Puffer, 0,02 m/l, pH 7,2 dialysiert. Anschließend erfolgt eine weitere Zentrifugation bei 10 000 x g, 15 min.

1.8.2 Chromatographie

Ein 1ml-Aliquot der vorbehandelten Ascitesflüssigkeit wird auf eine mit DEAE-Affi-Blue® (BIO-RAD) gepackte Säule aufgetragen (Bettvolumen 7 ml). Die Säule wird mit 20 ml Säulenpuffer (Tris-HCl, 0,02 m/l, pH 7.2) gewaschen. Die verschiedenen Proteine werden mit einem NaCl-Gradienten (0 - 100 mmol/l) bei einer Fließgeschwindigkeit von 30 - 40 ml/h eluiert. Fraktionen zu 1 - 2 ml werden gesammelt. Der Proteingehalt der einzelnen Fraktionen wird während der Elution ständig verfolgt. Diejenigen Fraktionen, die Antikörper enthalten, werden vereinigt und nach Zusatz von 0,02 % $NaN_3$ bei 4° C gelagert.

1.9 Screening-Tests zum Nachweis von Antikörpern in Vollblut, Zellkulturüberständen und Ascitesflüssigkeit

Zwei verschiedene Verfahren zum Auffinden von Antikörpern in Zellkulturüberständen wurden im Verlauf der Entwicklung des mAK gegen Digoxin angewandt, ein solid-phase Enzymimmunoassay und ein Radioimmunoassay. Letzterer erwies sich als besonders geeignet um schon im Screeningstadium mAK mit geringer Kreuzreaktion zu Digitoxin zu selektieren.

1.9.1 Solid-phase Enzymimmunoassay

Vorbereitung der ELISA-Platten

Der routinemäßige Screening-Test auf Antikörperproduktion wird als solid-phase Enzymimmunoassay (sp-ELISA)*durchgeführt. Die 96 Vertiefungen einer Microtiter®Platte werden zunächst mit dem auch bei der Immunisierung als Immunogen verwendeten Digoxin-BSA-Konjugat beschickt (51 µg/Loch) und 90 min.bei 37° C inkubiert. Die verbliebenen freien Bindungsstellen der Plastikoberfläche wegen mit Rinderserumalbumin (BSA) blockiert (0.5 % BSA, 0,05 % Tween 20, 0,02 % $NaN_3$ in PBS). Nach dreimaligem waschen mit Saline-Lösung (0,15 m NaCl, 0,05 % Tween-20, 0,02 % $NaN_3$ in aqua bidest) werden die so vorbehandelten Platten in einer feuchten Kammer bis zu 4 Wochen bei 4°C gelagert.

Durchführung des ELISA

In jede Vertiefung einer vorbeschichteten Platte wird ein Aliquot je eines Zellkulturüberstandes pipettiert. Nach Inkubation für 90 min bei 37°C wird der Inhalt der Vertiefungen verworfen, und die Platte dreimal mit Saline-Lösung gewaschen.

Zum Nachweis der gegebenenfalls an das Antigen (Digoxin-BSA) und damit an die feste Phase gebundenen Antikörper aus dem Zellkulturüberstand wird ein zweiter Antikörper (Ziegen anti-Maus Immun-

* ELISA = enzyme linked immunosorbent assay

globulin, anti-MIg, Fa. MEDAC, 0,25 µg/Loch) verwendet, der mit alkalischer Phosphatase als Nachweisreagenz markiert ist. Nach einer weiteren Inkubation (90 min, 37° C) wird durch Zugabe von Phosphatase-Substrat (p-Nitrophenylphosphat-Dinatriumsalz, SIGMA, 0,1 % in Diäthanolaminpuffer, pH 9,0, 100 µl/Loch) die Enzymreaktion gestartet. Nach etwa einer Stunde bei Raumtemperatur ist in denjenigen Vertiefungen der Platte, die Zellkulturüberstände von positiven Kulturen enthielten, eine deutliche Gelbfärbung erkennbar. Mit Hilfe eines speziell auf das Microtiter®-System abgestimmten 8-Kanal-Photometers (Titertek Multiskan, FLOW Laboratories) kann die Farbreaktion direkt in der Platte quantifiziert werden.

1.9.2 Radioimmunoassay zum Screening auf Antikörperproduktion

Der Screening-RIA wird als"Micro-RIA" (s.u.) in Mikrotiter-Platten durchgeführt. Ein Ansatz besteht aus:
100 µl Zellkulturüberstand
100 µl 125-J-Digoxin Tracer (DIAGNOSTIC PRODUCTS)
bzw. 100 µl 125-Digitoxin Tracer (DIAGNOSTIC PRODUCTS)
100 µl Normal-Humanplasma (Blutbank MAINZ)
Nach Inkubation bei Raumtemperatur (30 - 45 min) wird die freie und die an den Antikörper gebundene Radioaktivität durch Zugabe von 50 µl einer Suspension aus Dextran-behandelter Aktivkohle (MERCK) in Phosphatpuffer und anschließender Zentrifugation 1500 x g, 10 min getrennt. Die gebundene Radioaktivität (im Überstand) wird anhand eines aus jedem Loch der Microtiter®-Platte entnommenen Aliquots im Gamma-Szintillationszähler (KONTRON MR 480 c) gemesssen.

1.10 Radioimmunoassay für Digoxin

Der Digoxin-RIA wird ebenso wie der zuvor unter 3.9.2 beschriebene Screening-Test als "micro-RIA" in Microtiter®-Platten durchgeführt. Ein Ansatz besteht hier aus:
100 µl anti-Digoxin mAK
100 µl 125J-Digoxin-Tracer
100 µl Plasmaprobe
bzw.100 µl Digoxin-Standard in Plasma
Die Pipettierschritte (mit Ausnahme des Pipettierens der Einzelproben oder -Standards) werden mit 8- oder 12-Kanal Pipetten (TITERTEK®, FLOW Laboratories) ausgeführt.

Alle Inkubationszeiten und weiteren Arbeitsschritte entsprechen denjenigen des Screening-RIA.

1.11 Bestimmung der Immunglobulin-Subklasse des mAK

Der mAK wird in einem solid-phase-ELISA System näher charakterisiert.

Die Vertiefungen einer Mikrotiter®-Platte werden mit Antigen (Digoxin-BSA) beschichtet. Nach Inkubation dieser so vorbereiteten Platte mit dem mAK (2 h/37°C) aus der Zellkultur erfolgt ein zweiter Inkubationsschritt (1 h/37°C) mit anti-Maus-Immunglobuline verschiedener Klassen und Subklassen sowie mit verschiedenen Klassen leichter und schwerer Immunglobulin-Ketten. In einem weiteren Schritt wird ein enzymmarkiertes Ziegen-anti-Kaninchen-Immunglobulin zugesetzt. Nach der Inkubation von 1 h wird die Enzymreaktion durch Zugabe von p-Nitrophenylphosphat gestartet.

2. Ergebnisse

2.1 Immunantwort in der Maus

Vier Wochen nach Immunisierung kann bei 13 Tieren, aus einer Gruppe von 20, die Produktion von anti-Digoxin-Antikörpern radioimmunologisch im Vollblut nachgewiesen werden. Nur solche Tiere werden als Milzspender für die anschließenden Fusionsexperimente herangezogen, die sich besonders immunreaktiv zeigen.

Die Zunahme der Antikörperproduktion wird beispielhaft für zwei Tiere in Fig. 1 gezeigt.

2.2 Etablierung der Hybridom-Zellinie

Es wird ausschließlich die Zellinie P3X63.Ag8-635-Th als Fusionspartner verwendet. Hiermit lassen sich regelmäßig Fusionsfrequenzen (Prozentsatz der im HAT-Selektionsmedium überlebenden Zellpopulationen) von 50 - 80 % erreichen. (Tab. 1)

| Fusion Nr. | Anzahl proliferierender Kolonien nach HAT-Selektion | Fusionsfrequenz | Anzahl Kolonien mit spezifischer Antikörpersekretion | etablierte Hybridome |
|---|---|---|---|---|
| 17 | 314 | 81.7 % | 1 | 1 |
| 20 | 299 | 52,0 % | 3 | 1 |
| 28 | 448 | 77,8 % | 6 | 1 |

Tab. 1: Zusammenfassung der Fusionsausbeute am Beispiel dreier Fusionsexperimente. Fusion 17: 384 ursprünglich angesetzte Kulturen; Fusion 20 und 28: 576 ursprüngliche Kulturen.

Diejenigen Kolonien, die stabiles Wachstum und AK-Produktion aufweisen, werden nach der Methode des "Limiting dilution cloning" kloniert, d.h. die Zellen einer Kultur nach Fusion werden ausverdünnt und auf 2 Mikrotiter®-Platten (192 Einzelkulturen)so aufgesetzt, daß statistisch sich nur eine Zelle in jedem neuen Kulturgefäß befindet.

Nach 10 Tagen können in einzelnen Löchern in den Platten die ersten Zellklone makroskopisch festgestellt werden. Im mikroskopischen Bild ist in diesem Stadium das gleichmäßige und einheitliche Wachstum gut zu verfolgen. (Fig. 2)

Die Produktion von anti-Digoxin-Antikörpern durch die einzelnen Klone wird in regelmäßigen Abständen von 1 Woche zunächst mit Hilfe des solid-phase-ELISA, später mittels RIA überwacht.

Klone, die über mehrere Wochen stetig Antikörper produzieren, werden auf zwei verschiedenen Wegen expandiert. Zum einen werden Suspensionszellkulturen in größeren Petrischalen oder Zellkulturflaschen angelegt (in vitro-System).
Zum anderen werden die Hybridomklone im Peritoneum Pristan® vorbehandelter Mäuse als Ascites-Tumor (in vivo-System) propagiert. Zuvor wird jeweils ein Aliquot der Zellsuspension zur Aufbewahrung eingefroren.

2.3 Produktion von mAK in Zellkultur und Ascitesmaus

Über einen längeren Zeitraum hinweg wird die genetische Stabilität der Zellinien, und damit ihre Fähigkeit den gewünschten monoklonalen Antikörper zu erzeugen, in beiden Systemen dadurch überwacht, daß ständig der Antikörpertiter mittels RIA überprüft wird. Es zeigt sich, daß nur relativ wenige Klone in diesem Sinne stabil sind. Die genetische Instabilität der meisten Klone zeigt sich darin, daß der Antikörpertiter nach einigen Wochen ständig abnimmt, daß also die jeweilige. Ascitesflüssigkeit bzw. der jeweilige Zellkulturüberstand konzentrierter eingesetzt werden muß, um die gleiche Bindung des radioaktiven Tracers im RIA zu erzielen.

Von der Maus, in der die Hybridomlinie als Ascites-Tumor wächst, werden verschiedene Substanzen, vornehmlich Proteine und damit auch Immunglobuline sowie störende Enzyme (z.B. Proteasen) in die Ascitesflüssigkeit abgegeben.

Diese Komponenten stören bei einer späteren Verwendung im Testsystem, so daß eine Reinigung der Ascitesflüssigkeit daher wünschenswert erscheint.

2.4 Reinigung der Ascitesflüssigkeit

Die Reinigung der Ascitesflüssigkeit erfolgt affinitätschromatographisch über DEAE-Affi-Gel-Blue®.

Während der Elution mit einem NaCl-Gradienten (0 - 100 mMol) wird eine Abtrennnung der IgG-Frektion von störenden Proteasen - die erst bei NaCl-Konzentrationen >120 mMol/l eluiert werden - und von

Albumin erzielt (Fig. 3)
Der monoklonale anti-Digoxin-Antikörper wird im IgG-Peak bei 35 bis 50 mMol/l NaCl eluiert. Die Spitzenfraktionen (65 - 70) weisen dabei eine Proteinkonzentration von 80 µg/ml auf. Unter Verwendung eines Protease Test-Kits (BIO-RAD) kann nachgewiesen werden, daß die Fraktionen des IgG-Peaks frei von kontaminierten Proteasen sind.

In einem Bindungstest (RIA) zeigt sich, daß die Fraktionen mit der höchsten Proteinkonzentration im IgG-Peak nicht unbedingt gleichzusetzen sind mit denen. die höchste Affinität zu einem radioaktiven Digoxin-Tracer aufweisen.

2.5 Eigenschaften des mAK für Digoxin

2.5.1 Immunglobulin-Subklasse

Der anti-Digoxin-Antikörper mAK D28-A91-16-B64 (Kurzname: D50) ist ein Immunoglobulin der Subklasse $IgG_1$; die leichten Ketten sind dem Kappa-Typ zuzuordnen.

2.5.2 Affinität

Für den mAK D28A91-16-B64 wird die Affinitätskonstante für die Bindung an Digoxin anhand von Daten aus Radioimmunoassays bestimmt. Die Ergebnisse dieser Untersuchungen und der Vergleich mit den polyklonalen Antikörpern zweier kommerzieller RIA-Kits sind in Tab. 2 zusammengestellt.

| Bindungsreagenz | $K_a$ $^{-1}$ [molar $^{-1}$] |
|---|---|
| mAK D 50 | $4,1 \times 10^9$ |
| AS Diagnostic Products | $6,0 \times 10^9$ |
| AS Becton-Dickinson | $4,1 \times 10^9$ |

Tab. 2: Vergleich der $K_a$-Werte des mAK D50 mit den für Antiseren aus kommerziellen Kits angegebenen Daten. Die Bestimmung der Affinitätskonstante des mAK für Digoxin erfolgte aus RIA-Meßdaten.

Die gezeigten $K_a$-Werte weisen den anti-Digoxin mAK als hochaffinen Antikörper aus. Die Affinitätskonstante entspricht größenordnungsmäßig den von polyklonalen Antiseren bekannten Werten.

2.5.3 Sensitivität

Zur Ermittlung der Empfindlichkeit des auf dem mAK D 50 basierenden RIA-Systems werden Digoxin-Standards über einen Konzentrationsbereich von 0,03 ng/ml bis 100 ng/ml in Quadruplikaten gemessen. (Fig. 4)

Die Nachweisgrenze (im "micro-RIA") liegt hiernach deutlich unter 1 ng/ml. Hierin spiegelt sich die

hohe Affinität des mAK zu Digoxin wieder.

2.5.4 Spezifität

Wie bereits aus Fig. 4 ersichtlich wird, sind bei Mehrfachbestimmung nur sehr geringe Schwankungen für die Messwerte der Replikate zu verzeichnen. Beispielhaft für die reproduzierbare Assay-Präzision sind in Tab. 3 die Daten, aus denen die Standardkurve in Fig. 4 abgeleitet wurde, zusammen mit den jeweiligen Werten für die absolute und relative Standard-Abweichung angegeben.

| Digoxin [ng/ml] | % b/b (o) | Std.-Abw. absolut | Std.-Abw. relativ |
|---|---|---|---|
| 0,03 | 99,24 | 2,35 | 2,37 % |
| 0,05 | 98,53 | 3,03 | 3,08 % |
| 0,1 | 97,13 | 1,93 | 1,99 % |

**Tab. 3: Präzision des Digoxin-"micro-RIA"**
**(Angaben zu Testparametern s. Fig.4 )**

Gerade beim Einsatz eines anti-Digoxin-Antikörpers im Testsystem (z.B. zur Therapieüberwachung) ist es von entscheidender Bedeutung, wie gut der Antikörper Digoxin von ähnlichen Substanzen unterscheiden kann, die zum Teil endogen im Organismus vorhanden sind (Steroide) oder gleichzeitig mit der Digoxin-Medikation verabreicht werden (z.B. Spironolactone als Aldosteron-Antagonist).

Wünschenswert ist auch eine saubere Unterscheidung zwischen Digoxin und dem sich von diesem nur durch das Fehlen einer OH-Gruppe in Position 12 des Steroid-Ringsystems unterscheidenden Digitoxin.

Der mAK D 28-A91-16-B64 zeigt im "micro-RIA" eine hervorragende Spezifität für Digoxin (Fig. 5). Die Kreuzreaktion mit Digitoxin liegt bei 1,3 %, diejenige mit Spironolactone beträgt 0,007 %. Ebenso zeigen die verschiedenen getesteten Steroide nur geringfügige Interferenz (Fig. 5).

2.4.5 "micro-RIA" zur Digoxin-Bestimmung

Im Screening-Test hat sich das Microtiter-System hervorragend bewährt. Die Möglichkeit der Übertragung von Zellkulturüberständen in Testplatten mit der gleichen rastermäßigen Aufteilung erlaubt die Verwendung von speziellen Pipettiergeräten (12-Kanal-Pipetten) was in einer erheblichen Reduktion des Arbeits- und Zeitaufwands resultiert.

Aufgrund der guten Erfahrungen aus den Screeningassays werden auch für den eigentlichen Digoxin-Test die Vorteile des Microtiter-Rastersystems genutzt. Es wird der ursprünglich in herkömmlichen RIA-Reagenzröhrchen (Plastikreagenzgefäße, 75 x 12 mm, SARSTEDT) durchgeführte RIA an die Erfordernisse des Microtiter-Systems angepaßt.

Hierzu wird eine Reduktion des Assay-Volumens auf 300 µl vorgenommen. Die Trennung der gebundenen von der freien Radioaktivität wird durch eine geeignete Konzentration der Aktivkohlesuspension möglich, die zudem bei Anwendung ein zu pipettierendes Volumen von 50 µl nicht übersteigt.

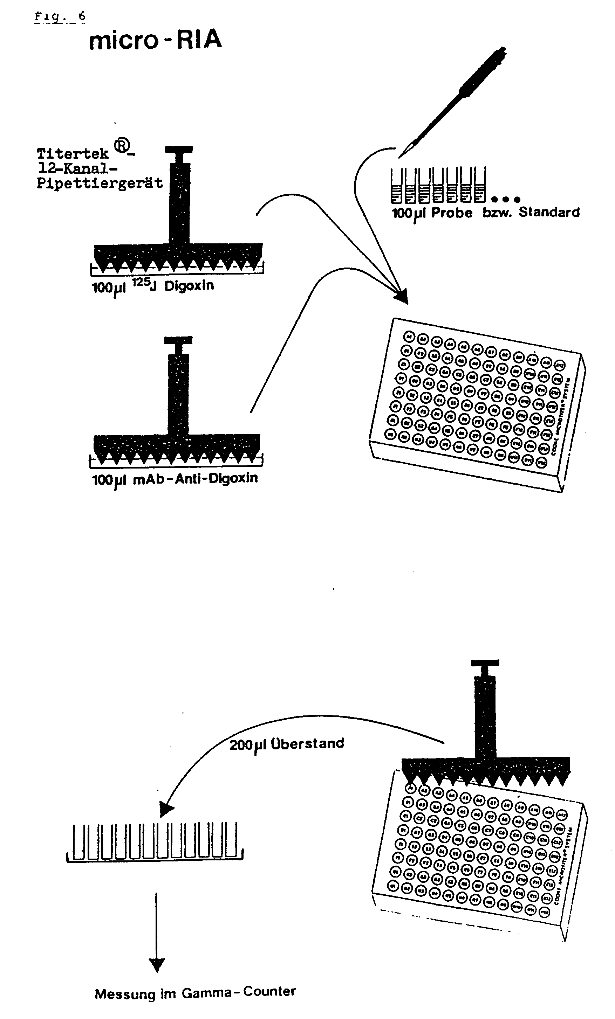

Es resultiert ein RIA-System, dessen Arbeitsweise schematisch in Fig. 6 dargestellt ist. Mit diesem System wurden alle RIA-Daten dieses Berichtes (Standardkurve, Spezifitätsnachweis etc.) erarbeitet.

Neben der deutlichen Einsparung an Arbeitszeit (im "micro-RIA" können ca. 300 Proben/Stunde verarbeitet werden) wird durch das geringe Assayvolumen auch die Menge der benötigten Reagenzien deutlich reduziert.

Die Zellinie MAX D5O (D28/A91/16/B64) wurde am 21. Dezember 1983 unter der Nummer I-272 bei der "Collection nationale de cultures de microorganismes (C.N.C.M), Institut Pasteur, Paris" gemäß Regel 28 des Europäischen Patentübereinkommens hinterlegt.

Anhang 1:

Zusammensetzung verwendeter Puffer-Lösungen und Kulturmedien

1. Puffer-Lösungen

1.1 Dulbecco's PBS (DPBS, Mengen in mg/l)

Literatur: EARLE, W.R. et al., J. Nat. Cancer Inst. 4, 165 (1943)

HANKS, J.H. und R.E. WALLACE, Proc. Soc. Exp. Biol. Med. 71, 196 (1949)

DULBECCO, R. und M. VOGT, J. Exp. Med. 99, 167, (1954)

| | PBS (DULBECCO) | EARLE's Salze | HANKS' Salze |
|---|---|---|---|
| NaCl | 8000 | 6800 | 8000 |
| KCl | 200 | 400 | 400 |
| $Na_2HPO_4$ | 1150 | - | 48 |
| $NaH_2PO_4 \cdot H_2O$ | - | 140 | - |
| $KH_2PO_4$ | 200 | - | 60 |
| $MgCl_2 \cdot 6H_2O$ | 100 | - | - * |
| $MgSO_4 \cdot 7H_2O$ | - | 200 | 200* |
| $CaCl_2$ | 100 | 200 | 140 |
| Glucose | - | 1000 | 1000 |
| Phenolrot | - | 10 | 10 |
| $NaHCO_3$ | - | 2200 | 350 |

* In der Originalzusammensetzung sind 100 mg/l $MgCl_2$ $6H_2O$ und 100 mg/l $MgSO_4$ $7H_2O$ angegeben.

1.2 Phosphat-Puffer (PBS, Mengen in g/l)

| | |
|---|---|
| 9.6 mM, pH = 7.4 | |
| NaCl | 8.0 |
| KCl | 0.2 |
| $Na_2HPO_4 \cdot 2H_2O$ | 1.44 |
| $KH_2PO_2$ | 0.2 |

1.3 Natriumhydrogencarbonat-Puffer

0.1 mol/l $Na_2HCO_3$ in aqua bidest mit
0.1 mol/l $NaCO_3$ in aqua bidest
auf pH = 9.0 eingestellt

1.4 Natrium-Acetat-Puffer (0.1 mol/l)

8.203 g $CH_3COONa$ } in 800 ml $H_2O$
29.22 g NaCl }
mit Essigsäure auf pH = 4.0 einstellen
ad 1 l aqua bidest.

1.5 Glycin-HCl-Puffer (0.1 mol/l)

Lsg. a: 0.1 mol/l Glycin (7.505 g/l) + 0.1 M NaCl (5.85 g/l)
Lsg. b: 0.1 mol/l HCl

Pfufferzusammensetzung: 88 % Lsg. a
12 % Lsg. b
pH = 3.2

1.6 Saline Waschlösung (für ELISA)

0.15 mol/l NaCl
0.05 % Tween 20
0.02 % $NaN_3$
in aqua. bidest.

1.7 Blockierungs-Puffer (für ELISA)

| | |
|---|---|
| 0.5 % HSA<br>0.05 % Tween 20<br>0.02 % $NaN_3$ | in PBS |

1.8 Diäthanolamin-Puffer (für ELISA)

48 ml Diäthanolamin
24.5 mg $MgCl_2$ (52.26 mg $MgCl_2 \cdot 6H_2O$) $l_2 \cdot 6H_2O$)
400 ml aqua bidest
mit 1 mol/l HCl einstellen auf pH = 9.0 ad 500 ml aqua bidest

2. Polyäthylenglycol-Lösung (zur Fusion)

20 g PEG 4000
20 min autoklavieren (121° C)
abkühlen auf 80° C
28 ml DPBS (mit 15 % DMSO) zugeben

## 3. Zellkultur-Nährmedien

### 3.1 Medium RPMI 1640 (Mengen in mg/l)

Literatur: MOORE, G.E. et al., J. Am. Med. Assoc. 199, 519 (1967)

| | |
|---|---|
| NaCl | 6000 |
| KCl | 400 |
| $Na_2HPO_4 \cdot 7H_2O$ | 1512 |
| $MgSO_4 \cdot 7H_2O$ | 100 |
| $Ca(NO_3)_2 \cdot 4H_2O$ | 100 |
| D-Glucose | 2000 |
| Phenolrot* | 5 |
| $NaHCO_3$ | 2000 |
| L-Arginin | 200 |
| L-Asparagin | 50 |
| L-Asparaginsäure | 20 |
| L-Cystin | 50 |
| L-Glutamin | 300 |
| L-Glutaminsäure | 20 |
| Glycin | 10 |
| L-Histidin | 15 |
| L-Hydroxyprolin | 20 |
| L-Isoleucin | 50 |
| L-Leucin | 50 |
| L-Lysin-HCl | 40 |
| L-Methionin | 15 |
| L-Phenylalanin | 15 |
| L-Prolin | 20 |
| L-Serin | 30 |
| L-Threonin | 20 |
| L-Tryptophan | 5 |
| L-Tyrosin | 20 |
| L-Valin | 20 |

| | |
|---|---|
| Glutathion | 1 |
| Biotin | 0,2 |
| Vitamin $B_{12}$ | 0,005 |
| D-CA-Pantothenat | 0,25 |
| Cholinchlorid | 3 |
| Folsäure | 1 |
| i-Inosit | 35 |
| Nicotinamid | 1 |
| p-Aminobenzoesäure | 1 |
| Pyridoxin · HCl | 1 |
| Riboflavin | 0,2 |
| Thiamin · HCl | 1 |

Flüssigmedium enthält 10 mg/l Phenolrot

zusätzlich:

| | |
|---|---|
| 0.002 mol/l | L-Glutamin |
| $10^5$ U/l | Penicillin-Streptomycin |
| $2 \times 10^{-5}$ mol/l | Mercaptoäthanol |
| 10 - 15 % | FCS |

3.2 HAT-Medium / HT-Medium

A: Aminopterin 3,82mg/200 ml aqua. bidest.

HT: Hypoxanthin 272,20 mg; Thymidin 76,50 mg — in 200 ml aqua. bidest.

HAT-Medium: 10 ml Stammlösung A + 10 ml Stammlösung HT auf 1000 ml RPMI 1640 (komplett mit Zusätzen)

HT-Medium: 10 ml Stammlösung HT auf 1000 ml RPMI 1640 (komplett mit Zusätzen)

3.3 Einfrier-Medium

70 % DPBS
10 % DMSO (Dimethylsulfoxid)
20 % FCS (fötales Kälberserum)

## Patentansprüche

1. Hybridzellinie, die monoklonale Antikörper mit höherer Affinität zum Digoxin als zum Digitoxin produziert, dadurch herstellbar, daß

a) man Mäuse mit Digoxin immunisiert und

b) daß man nach somatischer Zellfusion mit einer Maus-Tumorzellinie aus den hierdurch erhaltenen Zellkulturüberständen mit einem Radioimmunoessay (RIA) oder einem ELISA Assay diejenigen Hybride auswählt, die monoklonale Antikörper mit höherer Affinität zu Digoxin als zu Digitoxin bilden.

**2.** Hybridzellinie gemäß Anspruch 1, dadurch gekennzeichnet, daß eine Zellinie ausgewählt wird, die monoklonale Antikörper mit einer Kreuzreaktivität von etwa 1,3 % gegenüber dem Digitoxin produziert.

**3.** Hybridzellinie gemäß Anspruch 1, dadurch gekennzeichnet, daß die Selektionierung im Schritt b) zusätzlich mit Spironolacton durchgeführt und die Zellinie ausgewählt wird, die monoklonale Antikörper produziert, die hochaktiv gegen Digoxin und vernachlässigbar aktiv gegen Spironolacton ist.

**4.** Hybridzellinie gemäß Anspruch 3, dadurch gekennzeichnet, daß die Zellinie ausgewählt wird, die monoklonale Antikörper mit einer Kreuzreaktivität von weniger als 0,007 % gegenüber dem Spironolacton produziert.

**5.** Hybridzellinie MAK D50 (D28/A91/16/B64), hinterlegt beim Institut Pasteur mit der Hinterlegungsnummer I-272.

**6.** Monoklonaler Antikörper mit höherer Affinität zum Digoxin als zum Digitoxin, dadurch herstellbar, daß eine Hybridzellinie nach einem der Ansprüche 1 bis 4 propagiert wird und die monoklonalen Antikörper isoliert werden.

**7.** Monoklonaler Antikörper gemäß Anspruch 6, dadurch gekennzeichnet, daß er eine Kreuzreaktivität von etwa 1,3 % gegenüber dem Digitoxin aufweist.

**8.** Monoklonaler Antikörper gemäß Anspruch 6 oder 7, dadurch gekennzeichnet, daß er eine Kreuzreaktivität von weniger als 0,007 % gegenüber dem Spironolacton aufweist.

**9.** Verfahren zur Herstellung einer Hybridzellinie gemäß Anspruch 1 bis 4, bei dem man

a) Mäuse mit Digoxin behandelt,

b) Milzzellen dieser Mäuse mit Mausmyelomzellen fusioniert,

c) Hybride von nicht fusionierten Zellen abtrennt,

d) und daß man aus den Zellkulturüberständen diejenigen Hybride auswählt, welche die im Anspruch 1, 2, 3 bzw. 4 angegebenen Affinitäten besitzen.

**10.** Verfahren zur Herstellung monoklonaler Antikörper gemäß Anspruch 6 bis 8, bei dem man die Hybridzellen gemäß den Ansprüchen 1 bis 5 in vitro oder in vivo züchtet und die monoklonalen Antikörper isoliert.

**11.** Verwendung des monoklonalen Antikörpers nach Anspruch 6, 7 oder 8 zum Nachweis, zur in vitro Diagnose oder zur quantitativen Bestimmung des Digoxins.

**12.** Verwendung des monoklonalen Antikörpers nach Anspruch 11, bei der Nachweis, Diagnose oder quantitative Bestimmung am Vollblut erfolgen.

**13.** Testsystem, dadurch gekennzeichnet, daß es neben dem monoklonalen Antikörper gemäß Anspruch 6, 7 oder 8 einen markierten Digoxin Tracer enthält.

**14.** Testsystem gemäß Anspruch 13, dadurch gekennzeichnet, daß als Tracer $^{125}$J-Digoxin verwendet wird.

**Claims**

**1.** Hybrid cell line which produces monoclonal antibodies having a greater affinity for digoxin than for digitoxin, capable of being prepared by

a) immunising mice with digoxin and

b) after somatic cell fusion with a mouse tumour cell line, selecting from the resultant cell culture

supernatants, by radioimmunoassay (RIA) or by ELISA assay, those hybrids which form monoclonal antibodies having a greater affinity for digoxin than for digitoxin.

**2.** Hybrid cell line according to claim 1, characterised in that a cell line is selected which produces monoclonal antibodies having a cross-reactivity of about 1.3% in relation to digitoxin.

**3.** Hybrid cell line according to claim 1, characterised in that the selection in step b) is additionally carried out using spironolactone and the cell line which is selected is one which produces monoclonal antibodies which are highly active against digoxin and negligibly active against spironolactone.

**4.** Hybrid cell line according to claim 3, characterised in that the cell line selected is one which produces monoclonal antibodies having a cross-reactivity of less than 0.007% in relation to spironolactone.

**5.** Hybrid cell line MAK D50 (D28/A91/16/B64), deposited at the Institut Pasteur under Serial Number I-272.

**6.** Monoclonal antibody having a greater affinity for digoxin than for digitoxin, which can be prepared by propagating a hybrid cell line according to one of claims 1 to 4 and isolating the monoclonal antibodies.

**7.** Monoclonal antibody according to claim 6, characterised in that it has a cross-reactivity of about 1.3% in relation to digitoxin.

**8.** Monoclonal antibody according to claim 6 or 7, characterised in that it has a cross-reactivity of less than 0.007% in relation to spironolactone.

**9.** Process for preparing a hybrid cell line according to claims 1 to 4, wherein
a) mice are treated with digoxin,
b) spleen cells from these mice are fused with mouse myeloma cells,
c) hybrids are separated from unfused cells,
d) and, from the cell culture supernatants, those hybrids are selected which have the affinities recited in claim 1, 2, 3 or 4.

**10.** Process for preparing monoclonal antibodies according to claims 6 to 8, wherein the hybrid cells according to claims 1 to 5 are cultured in vitro or in vivo and the monoclonal antibodies are isolated.

**11.** Use of the monoclonal antibody according to claim 6, 7 or 8 for the detection, in vitro diagnosis or quantitative determination of digoxin.

**12.** Use of the monoclonal antibody according to claim 11 wherein the detection, diagnosis or quantitative determination are carried out on whole blood.

**13.** Test system, characterised in that it contains, in addition to the monoclonal antibody according to claim 6, 7 or 8, a labelled digoxin tracer.

**14.** Test system according to claim 13, characterised in that $^{125}$I-digoxin is used as the tracer.

**Revendications**

**1.** Lignée cellulaire hybride qui produit des anticorps monoclonaux à plus grande affinité pour la digoxine que pour la digitoxine, qui peut être préparée en ce que
a) l'on immunise des souris avec de la digoxine et
b) après fusion cellulaire somatique avec une lignée cellulaire tumorale de souris l'on choisit, à partir des surnageants de culture cellulaire ainsi obtenus, au moyen d'un test radioimmunologique (RIA) ou d'un test ELISA, les hybrides qui forment des anticorps monoclonaux à plus grande affinité pour la digoxine que pour la digitoxine.

**2.** Lignée cellulaire hybride selon la revendication 1, caractérisée en ce que l'on choisit une lignée cellulaire qui produit des anticorps monoclonaux ayant une réactivité croisée d'environ 1,3% vis à vis

de la digitoxine.

3. Lignée cellulaire hybride selon la revendication 1, caractérisée en ce que la sélection à l'étape b) est réalisée en outre avec la spironolactone et la lignée cellulaire choisie est celle qui produit des anticorps monoclonaux qui sont très actifs contre la digoxine et qui ont une activité négligeable contre la spironolactone.

4. Lignée cellulaire hybride selon la revendicaticn 3, caractérisée en ce que la lignée cellulaire choisie est celle qui produit des anticorps monoclonaux ayant une réactivité croisée inférieure à 0,007% vis à vis de la spironolactone.

5. Lignée cellulaire hybride MAK D50 (D28/A91/16/B64) déposée auprès de l'Institut Pasteur sous le numéro de dépôt I-272.

6. Anticorps monoclonal à plus grande affinité pour la digoxine que pour la digitoxine, qui peut être obtenu en ce qu'une lignée cellulaire hybride selon l'une des revendications 1 à 4 est propagée et les anticorps monoclonaux sont isolés.

7. Anticorps monoclonal selon la revendication 6, caractérisé en ce qu'il présente une réactivité croisée d'environ 1,3% vis à vis de la digitoxine.

8. Anticorps monoclonal selon la revendication 6 ou 7, caractérisé en ce qu'il présente une réactivité croisée inférieure à 0,007% vis à vis de la spironolactone.

9. Procédé de préparation d'une lignée cellulaire hybride selon les revendications 1 à 4, dans lequel
   a) on traite des souris à la digoxine,
   b) on fusionne des cellules spléniques de ces souris avec des cellules de myélome de souris,
   c) on sépare les hybrides des cellules non fusionnées,
   d) et on choisit à partir des surnageants de culture cellulaire les hybrides qui possèdent les affinités indiquées dans la revendication 1, 2, 3 ou 4.

10. Procédé de préparation d'anticorps monoclonaux selon les revendications 6 à 8, dans lequel on cultive les cellules hybrides selon les revendications 1 à 5 in vitro ou in vivo et on isole les anticorps monoclonaux.

11. Utilisation de l'anticorps monoclonal selon la revendication 6, 7 ou 8 pour la mise en évidence, le diagnostic in vitro ou la détermination quantitative de la digoxine.

12. Utilisation de l'anticorps monoclonal selon la revendication 11 pour la mise en évidence, le diagnostic ou la détermination quantitative sur du sang total.

13. Système de test, caractérisé en ce qu'il contient un traceur de digoxine marqué en plus de l'anticorps monoclonal selon la revendication 6, 7 ou 8.

14. Système de test selon la revendication 13, caractérisé en ce que la $^{125}$I-digoxine est utilisée comme traceur.

Anhang 2

Fig. 1

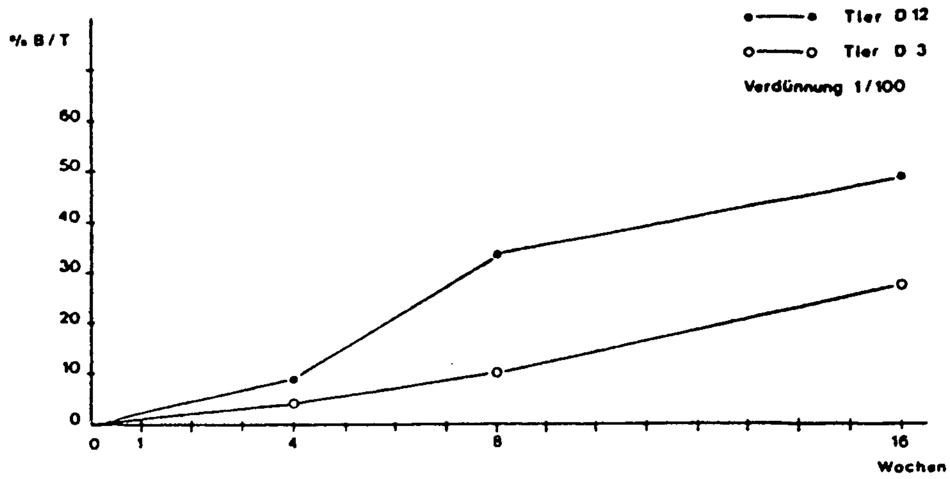


Fig. 2

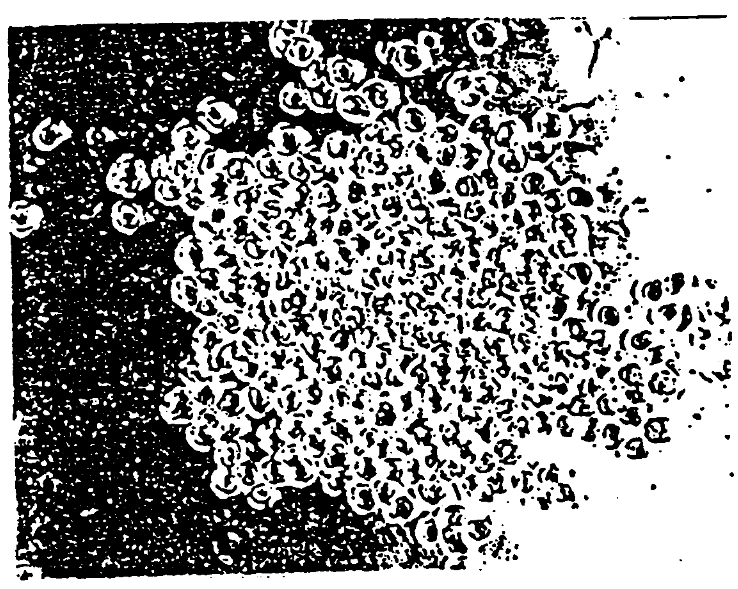

Fig. 3

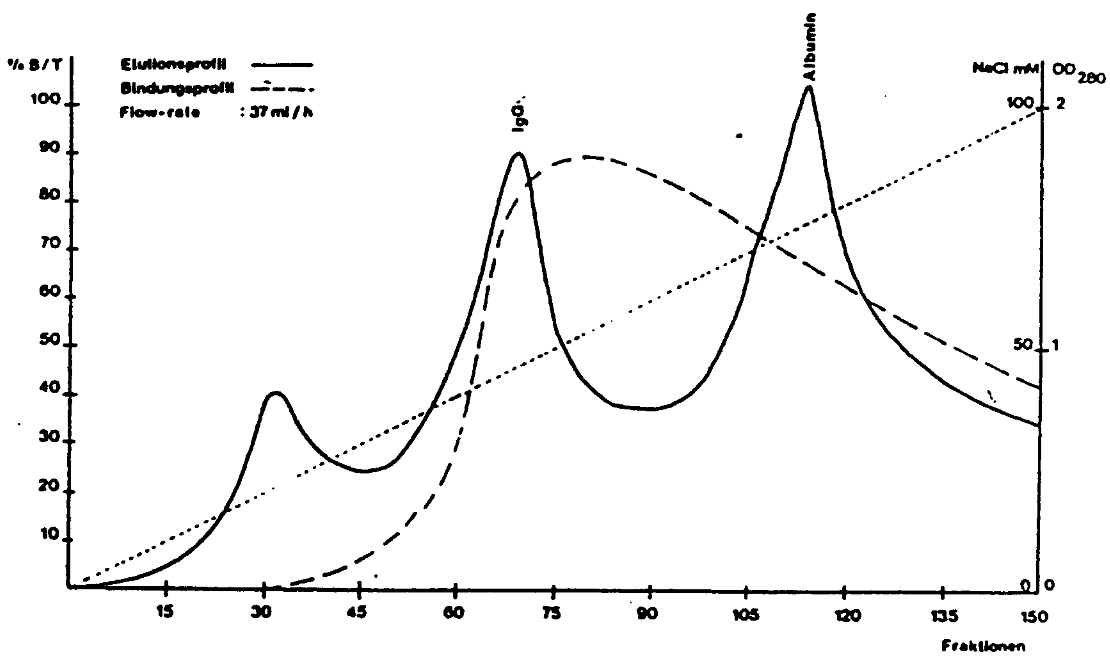
% B/T
Elutionsprofil
Bindungsprofil
Flow-rate : 37 ml/h
IgG
Albumin
NaCl mM
$OD_{280}$
100
90
80
70
60
50
40
30
20
10
15
30
45
60
75
90
105
120
135
150
Fraktionen
100 2
50 1
0 0

Fig. 4

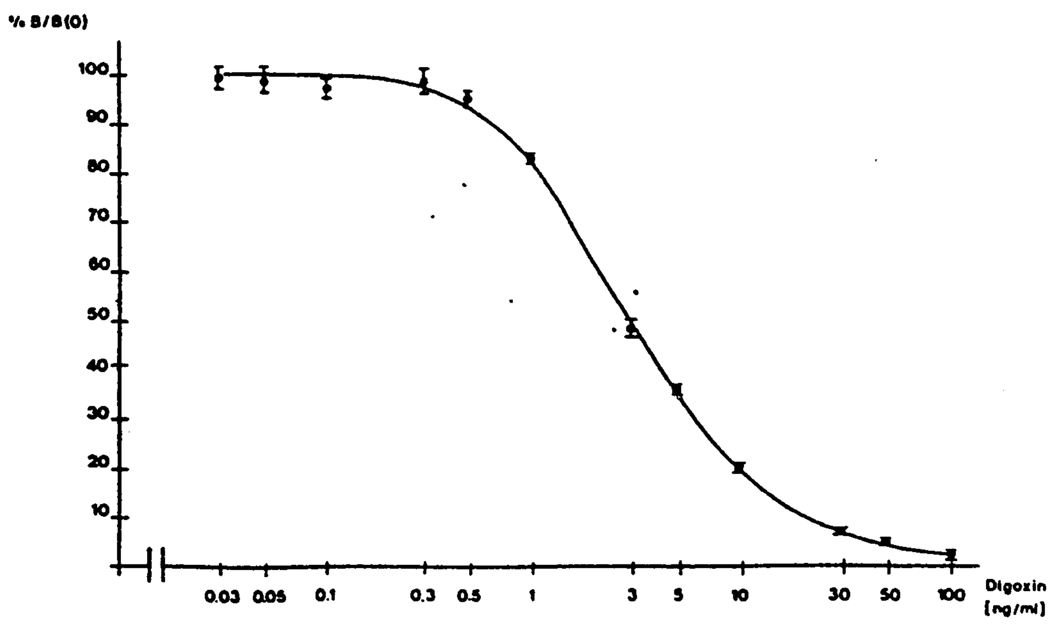
% B/B(0)
100
90
80
70
60
50
40
30
20
10
0.03
0.05
0.1
0.3
0.5
1
3
5
10
30
50
100
Digoxin
[ng/ml]

Fig. 5

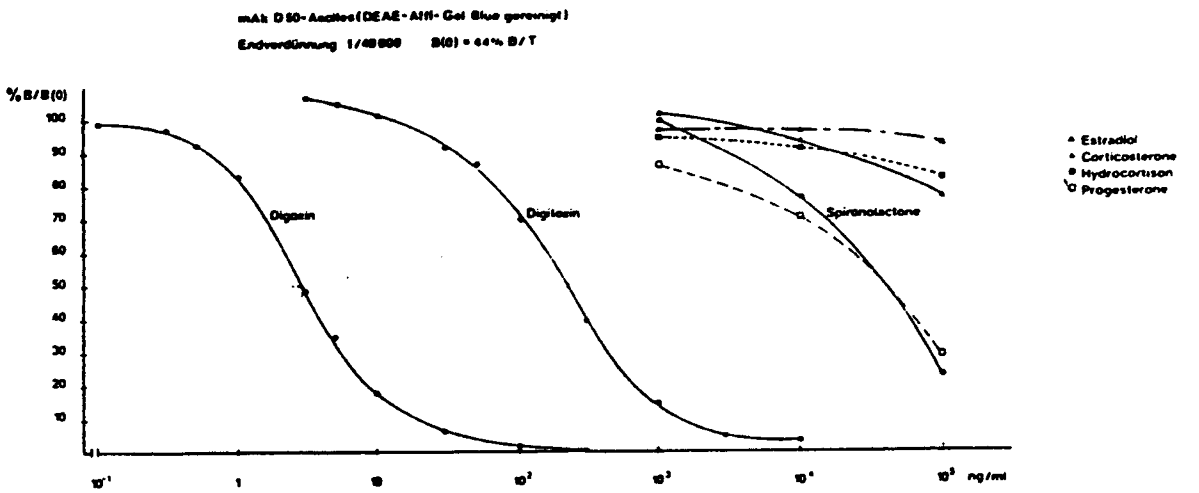
%B/B(0)
100
90
80
70
60
50
40
30
20
10
Digoxin
Digitoxin
Spironolactone
Estradiol
Corticosterone
Hydrocortison
Progesterone
1
10
ng/ml

Fig. 6

# micro-RIA